# EUROPEAN PATENT APPLICATION

(11) **EP 3 335 575 A1**
(43) Date of publication of application: **20.06.2018**
(21) Application number: 16835522.0
(22) Date of filing: 28.07.2016
(51) Int. Cl.: A23L 29/30

(54) **METHOD AND PROCESS OF ENRICHMENT OF AN AGAVE FRUCTAN IN A PREBIOTIC DRINK**

(30) Priority: 10.08.2015 MX 2015010290
(71) Applicant: Bebidas Prebióticas Suin, S.A.P.I. de C.V., 76160 Santiago de Querétaro, Querétaro (MX)
(72) Inventor: MUHLBACH CÁCERES, Paulina, 76160 Santiago de Querétaro, Querétaro (MX); REYES CERVANTES, Vicente, 76160 Santiago de Querétaro, Querétaro (MX)
(74) Representative: Villa, Livia
(86) International application number: PCT/MX2016/050011
(87) International publication number: WO 2017/026883

(57) **Abstract**

The process of enrichment of a single fructan from a mixture of *Agave Tequilana Weber* from Jalisco and *Agave Angustifolia Haw* from Oaxaca with agave syrup showed that the innate prebiotic characteristics thereof are not affected by drink enrichment. The method of enrichment developed shows that the degree of polymerisation is not affected, to such an extent that the analyses show the same quantity added to the drink using the HPAEC-PAD method. The method of enrichment was also validated *in vitro,* repeating its prebiotic benefit in 4 *bifidobacteria* strains and 4 *lactobacilli* strains, and *in vivo* in mice.

## Description

### Field of the invention.

This invention relates to the development of a method and process of enrichment of a fructan of *Agave Tequilana Weber* and Oaxacan *Agave Angustifolia Haw* in a prebiotic drink with unique characteristics that offers the prebiotic effect in probiotic and animal strains, using the in vitro and in vivo method. The developed process of enrichment protects the unique fructan against the polimerization grade change, thus allowing the preservation of its innate prebiotic characteristics.

### Historical background.

Mexico is one of the countries in Latin America most affected by gastrointestinal problems. The National Institute of Statistics and Geography (INEGI) estimates that 5 million people suffer from gastrointestinal infections per year and that 5% of yearly deaths in this country are due to this cause. (1)

Furthermore, more than 64% of the population suffers from some digestive problem, such as gastritis, colitis, esophageal reflux, irritable colon, among others. (1) Adding to this fact, lactose intolerance is a significant health issue around the world, which arises from the incapacity of the intestine to digest lactose due to a deficiency of the lactase enzyme in the small intestine. This condition can affect the quality of life of any age group. (2) In Mexico, the prevalence by area is 30% in the central area, 22% southeast, and 9% in the country's north; there is no link to any age group or sex. (3)

The immune system is responsible for protecting the body against different organisms that may affect the human body system. The digestive tract is directly linked, as approximately 70% of the cells of the immune system are found in the intestine. The intestine must have the capacity to distinguish between good bacteria (probiotics) and bad bacteria. Good bacteria prevent the excessive growth of yeasts or parasites. Microflora must be strong in order to be able to help improve the function of the cells of the immune system. (4)

Among others, a person's lifestyle is a significant factor and can lead to an increase in certain illnesses. Carbohydrates are essential for all living
organisms, and they are the most abundant class of biological molecules. Most of the carbohydrates present in nature are polysaccharides, which give rise to monosaccharides or derivatives when submitted to acid or enzymatic hydrolysis. Due to their biological function, polysaccharides can be divided into two main groups, structural and storage. Among storage polysaccharides of vegetable origin are starch and fructans. (5) Carbohydrates can be classified as digestible or indigestible, depending on the type of linking present in the structure. Due to the configuration of the anomeric C2 in their fructose monomers, fructans are resistant to hydrolysis by human digestive enzymes (b-glucosidase, maltase-isomaltase, sucrose, among others), owing to which those carbohydrates are classed as "indigestible". (6)

There is strong scientific evidence regarding the role of type b(2-1) fructans such as prebiotics. A prebiotic is defined as an indigestible dietary ingredient which produces a beneficial effect on the host by stimulating the selective growth and/or metabolic activity of a limited number of bacteria in the colon" (). Due to the important characteristics of fructans, to which prebiotic properties may be attributed. Fructans are capable of selectively stimulating the growth of *Bifidobacteria* or *Lactobacilli* at the expense of putrefactive bacteria; furthermore, the fermentation of said fructans gives rise to the production of acetate, propionate, and butyrate, classed as short-chain fatty acids (SCFA) and lactic acid, which contribute to the normal function of the large intestine. (7)

Inulin is one of the 5 fructan groups and it is found in high quantities in foods such as artichoke and asparagus; it is fermented by the microflora in the colon and acts as soluble fiber. (7) It is of great interest that they selectively stimulate the growth of bifidobacteria at the expense of bacteroides, *Clostridium,* or coliform bacteria, which remain at low levels. (7).

The bacterial fermentation of these prebiotics produces SCFA in the colon, including small quantities of butyric acid, which have been shown to increase apoptosis in the colon. (7)

A recent study on fructans in agave mentions high levels of these prebiotics in the *Agavaceae* family. (8)

There is an enormous interest throughout the country regarding the use of ethnic or endemic resources, but in recent years this interest has increased to integrally use different agave species, of which the greater part are not used to elaborate alcoholic drinks like tequila, mezcal, bacanora, and sotol.

Regardless, at present a large number of farm workers have increased their agave cultivation, including species protected by the Appellation of Origin used to elaborate the previously mentioned drinks. This has generated some concern in recent years for the need to find other uses for said cultivation of all species found in the country, but mainly of *Agave Angustifolia Haw* from the state of Oaxaca and the blue variant of *Agave tequilana Weber* from the state of Jalisco.

The enrichment of a drink with Oaxacan *Agave angustifolia Haw* fructans as well as syrup made from agave from the same region presents very high potential, not only in the technological aspect but also economically and socially for the farm workers and merchants of this crop. Additionally, it would pinpoint and spread awareness of the agave fructans of this region, which would have a technological impact not only for this company but also for agave cultivators and the country in general.

### Description of figures.

Figure 1: Samples sent for analysis B1-B5.
Figure 2: Chromatographic profile of carbohydrates and fructanes in the samples B1-B5 (The arrows indicate the different grades of polimerization)
Figure 3: Thin-layer chromatography for identification of fructans in prebiotic drinks enriched with Blend
Figure 4: Levels of fructose, glucose, sucrose, kestose, DP=5, and total carbohydrates in the prebiotic drinks
Figure 5: Growth plates of the prebiotic drink probiotics
Figure 6: Cage with mouse of the prebiotic drink in the vivarium

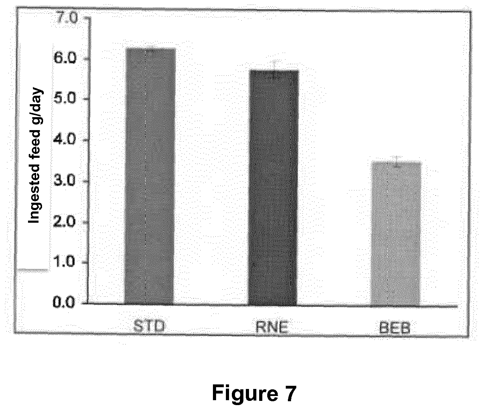

Figure 7: Differences shown between ingestion of the feed of the three groups of mice (STD Control, RNE Chicory Inulin, BEB drink)

### Description of the invention.

With the goal of fostering a healthy lifestyle through the consumption of functional products with natural ingredients as a base, throughout the years 2012-2014 an enrichment process has been developed for a prebiotic drink made from a mix of fructans that originate from *Agave Tequilana Weber* from Jalisco and/or *Agave Angustifolia Haw* from Oaxaca and agave syrup. This enrichment achieves a prebiotic effect, which has been evaluated *in vivo* and *in vitro.*

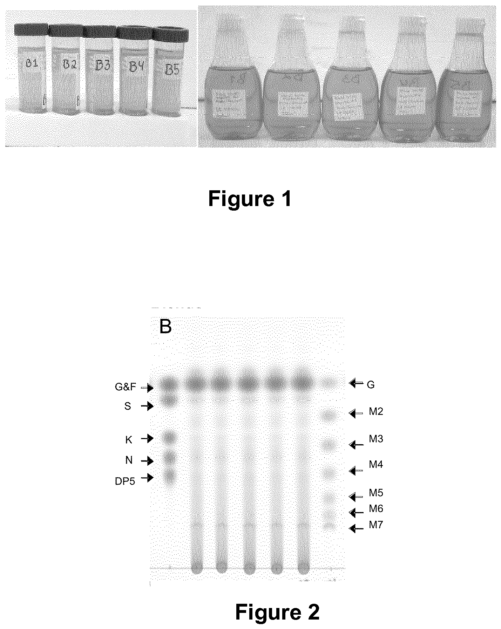

First, the ideal mixture of fructan + agave syrup was sought; 5 different blends were analyzed. These were stored at room temperature and their appearance and consistency were analyzed throughout the storage period. The samples did not exhibit changes in appearance and consistency during a period of 70 days of storage. The analyzed blends (Figure 1) exhibited a content of soluble solids that was similar among the samples (73.48 to 73.80 °Brix). The sample blends exhibited homogeneity, presenting pH values of 5.2; that is to say, a slightly acidic pH level. The color of the samples was determined through absorbance at 560 nm in a Biorad Benchmark/Plus Microplate spectophotometer (9) for the agave syrup, using glycerol as reference.

To determine the ideal blend, the following were analyzed:
1) Content of soluble carbohydrates, by an enzymatic method in single samples of inulin (5 samples), honey (5 samples), and blend; this in order
   to assure that the blend was the best option to be used as an ingredient to enrich the drink. The blend samples exhibited a content of fructans in a range of 328 to 347 mg/g.
2) Carbohydrates profile by TLC: solutions were prepared at a concentration of 50 mg/mL. One microliter (µL) of each solution was taken and analyzed by thin-layer chromatography, along with oligosaccharide standards (MOS and FOS). Depending on the staining used, it is possible to differentiate the glucose and fructose derivatives according to the color of the spot (blue and reddish, respectively). The samples exhibited low DP fructans (fructans DP3-DP10) and high DP fructans (fructans DP>10). Additionally, the presence of a small blue spot was observed below the maltose corresponding to neokestose (trisaccharide base of neoseries-type fructans). The abundant spot on the base line of the TLC plate corresponds to fructans (polymers with DP>10 units); therefore it can be assumed that the analyzed samples contain fructans with extensive DP (Figure 2).

Once the ideal blend was obtained, the enrichment method was developed for the prebiotic drink, which was evaluated *in vivo* and *in vitro* to determine the prebiotic effect.

The enrichment was performed through a method of adding the blend in exact quantities and under controlled temperatures. This method is based on the control of the grades of polymerization of the blend and its fructans. This is so the structure is not damaged and to achieve the prebiotic benefit.

Water previously submitted to a resin filtering process, activated carbon, and softening filter in which the water must contain no more than 30 ppm of calcium carbonate were mixed at 45 °C with the blend, at a proportion of 11:1 respectively. This allowed the blend fructans to become soluble and dispersed in a very large aqueous base, which in turn allowed resistance to the pasteurization temperature of 60 °C for 30 minutes so that the structure was not damaged and the prebiotic benefit was retained. Pasteurization was performed in a cauldron with heating by gas and resistances. The mixture was constantly stirred at no more than 60 rpm with a propeller stirrer. Afterwards, the mixture was stirred as it cooled without any sort of cold exchange, as fructans undergo structural changes if exposed to sudden temperature changes. It was necessary to pay close attention to the operating conditions so the enriched mixture would not be contaminated (isolated cauldron in a fresh area at no more than 28 °C).

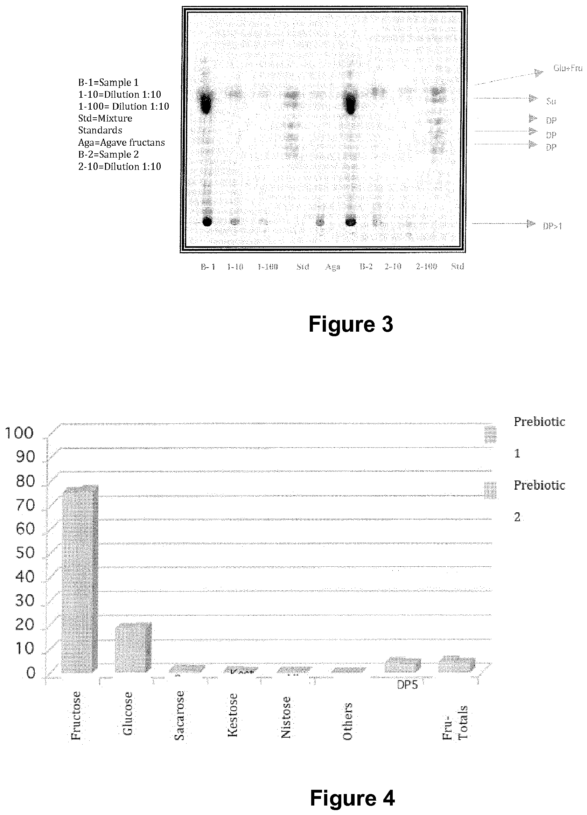

To determine whether the developed enrichment process was ideal, TLC analysis was performed on the beverage, which determined the presence of fructans and their profile in the enriched prebiotic drink. The presence of fructans was so high that the drink underwent dilutions. The presence of fructans with a DP higher than 10 was observed. (Figure 3) The quantity of fructans was determined by the enzymatic technique, which showed that the drinks obtained 3.7-4% of fructans. This proves the success of the enrichment process, as 4% of fructans was added to the drink. To correct this value, carbohydrates were individually measured by liquid chromatography (HPAEC-PAD); a value of 3.9% of fructans was exhibited (Figure 4).

To achieve a scientific evaluation and validity for this enrichment process of the blend in the prebiotic drink, a validation was performed with *in vitro* studies using different prebiotic strains, and *in vivo* using laboratory mice for the study.

Within the inulin *in vitro* evaluations, readings by thin-layer chromatography and high-performance anion-exchange chromatography coupled to an amperometric detector for the detection of fructans were performed. As well, the total of soluble solids was measured in the drink in °Brix. Finally, the quantity of present fructans was quantified through an enzymatic method.

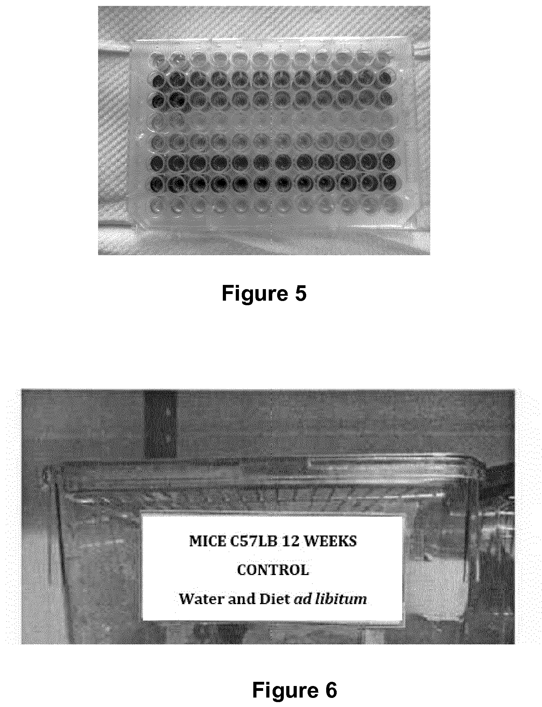

Through *in vitro* evaluations, the prebiotic potential of the drinks was determined by the probiotic bacteria study, using the Man, Rogosa, and Sharpe culture medium, also known as the MRS medium. (Figure 5) The probiotic bacterias studied were of the bifidobacteria (six strains) and Lactobacilli (four strains) types. Of the Bifidobacterium genus: *B*. *adolescentis* (ATCC 15703), *B. animalis* (27536), *B. bifidum* (ATCC 29521), *B. breve* (ATCC, 15700), *B. longum* (15707), *B. infantis* (ATCC 25962); and four strains of the Lactobacillus genus: *L. acidophillus* (ATCC 4356), *L. casei* (ATCC 393), *L. paracasei* (ATCC 25302), and *L. rhamnosus* (ATCC 53103).

It was concluded that the prebiotic effect of the enrichment process of the drinks exists. There was growth in:
1) 4 strains of the *Bifidobacterium* genus: *B. adolescentis* (ATCC 15703), *B. animalis* (27536), *B. bifidum* (ATCC 29521), *B. infantis* (ATCC 25962)
2) 4 strains of the *Lactobacillus* genus: *L. acidophillus* (ATCC 4356), *L. casei* (ATCC 393), *L. paracasei* (ATCC 25302), and *L. rhamnosus* (ATCC 53103).

The *Bifidobacteria longum* and *Bifidobacteria breve* probiotics did not grow in any of the mediums, regardless of the fact that it was attempted several times.

The *in vivo* study was performed with ten and eight mice of the C57B|/6J line from the vivarium at Zacatenco, Mexico, aged 12 weeks. They were used at the beginning of the experiment, under controlled temperature and humidity and a light/dark cycle of 12 hours. (Figure 6) The amount of ingested feed was measured weekly, as well as water consumption, mice weights, feces accumulation (residual carbohydrates were measured), and blood samples were taken from the mice's tails to determine glucose. After 5 weeks, the mice were put down and the large intestines and colon contents were collected to measure pH and SCFA. Additionally, blood was taken from the portal veins to determine triglycerides (TGA) and cholesterol.

The results showed the following:
1) The mice that consumed the prebiotic drink ate less (due to the fiber in the drink and their feeling of fullness)
   Figure 7
2) The mice that consumed the prebiotic drink showed a smaller content of triglycerides and cholesterol than the other two groups, but the difference is not major.
3) The feces of the BEB mice show a larger content of residual carbohydrates than the other two groups and a larger production of SCFAs.
4) The prebiotic drink achieves the prebiotic effect within the digestive system of the mice.

To conclude, the experimental design tests for the enrichment process of the drink were performed, as well as the ideal blend development, in the pilot production plant for the prebiotic drink. The technological project had an execution period of 24 months in order to favor the technical enrichment process of a drink with a single fructan, thus achieving its innate benefit for those consuming the prebiotic drink.

It is important to mention that the drink possesses unique characteristics of pH and acidity to maintain the shelf life of the contained fructan during the shelf life of the product.

### Bibliographic references.

1. INEGI. 1996.
2. Lactose Intolerance. How is Lactose Intolerance Diagnosed?
   The National Digestive Diseases Information Cleaninghouse
   (NDDIC). Available at: www.digestive.niddk.nih.gov
3. Lopez P. y cols. Lactose maldigestion. Definition, prevalence in Mexico and it's implications in milk consumption. Rev. invest. Clin: 48 (Supl): 15-22, no. 1996
4. The Blokehead. Súper Inmunidad y Súper Alimentos. Capítulo 3: Centros Inmunológicos del Cuerpo. 2015. Babelcube Inc.
5. Hicks, J.J. 1995. Carbohidratos: estructura, configuración y propiedades. En: Bioquímica. Mc Graw Hill. 142-161
6. Roberfroid, M. B. 2000. Chicory fructooligosaccharides and the gastrointestinal tract. Nutr. 16: 677-679.
7. Gibson, G.R. y Roberfroid, M.R. 1995. Dietary modulation of the human colonic microbiota: introducing the concept of prebiotics. J. Nutr. 125: 1401-1412.
8. López, M.G., Mancilla-Margalli, N.A. y Mendoza-Díaz, G. 2003. Molecular structure of fructans from Agave tequilana Weber var, Azul. J. Agric. Food Chem. 51: 7835-7840.
9. USA a la Norma Mexicana NMX-FF-110-SCFI-2008

## Claims

1. A unique method and process to enrich a prebiotic drink with unique characteristics.

2. The unique fructan (blend of *Agave Angustifolia Gaw* and *Agave Tequilana Weber* + agave syrup) presented a high content of soluble carbohydrates between 328 to 347 mg/g (through an enzymatic method) with a complex profile of low DP (fructans DP3-DP10) and high DP fructans (fructans DP>10), as well as the presence of neokestose (trisaccharyde base of neoseries-type fructans) using the TLC method.

3. The method includes protection of the blend's (fructan) degree of polymerization so its innate prebiotic capacity is intact in the prebiotic drink and its prebiotic effect can reach the consumers.

4. With the developed enrichment method, it was proven that the drink has the prebiotic effect in vitro in: 4 strains of the Bifidobacterium genus: *B*. *adolescentis* (ATCC 15703), *B. animalis* (ATCC 27536), *B. bifidum* (ATCC 29521), *B. infantis* (ATCC 25962), and 4 strains of the Lactobacillus genus: *L. acidophillus* (ATCC 4356), *L. casei* (ATCC 393), *L. paracasei* (ATCC 25302), and *L*. *rhamnosus* (ATCC 53103).

5. With the developed enrichment method, using *in vivo* validation, the mice exhibited a smaller content of triglycerides and cholesterol, higher content of residual carbohydrates in the feces, and a higher production of SCFAs.

6. The drink possesses unique pH and acidity characteristics that protect the prebiotic capacity of the drink during the shelf life, aside from the development of the enrichment method/process.
